# EUROPEAN PATENT APPLICATION

(11) **EP 2 012 246 A1**
(43) Date of publication of application: **07.01.2009**
(21) Application number: 07740056.2
(22) Date of filing: 28.03.2007
(51) Int. Cl.: G06F 19/00, G06Q 10/00, C12N 15/09

(54) **METHOD OF PREDICTING THE SECONDARY STRUCTURE OF RNA, PREDICTION APPARATUS AND PREDICTION PROGRAM**

(30) Priority: 28.03.2006 JP 2006088599
(71) Applicant: Nec Soft, Ltd., Tokyo 136-0082 (JP)
(72) Inventor: AKITOMI, Jou, Tokyo 136-0082 (JP)
(74) Representative: Glawe, Ulrich
(86) International application number: PCT/JP2007/056618
(87) International publication number: WO 2007/116787

(57) **Abstract**

The present invention is to provide a method for predicting secondary structure of RNA capable of predicting the secondary structure which has been difficult to predict the secondary structure including pseudonot structure, and an apparatus for predicting secondary structure of RNA using the method for predicting. The method for predicting secondary structure of RNA according to the present invention is **characterized in that**:
A method for predicting secondary structure of RNA comprising the steps of:
searching base capable of forming a stem structure from the RNA sequence to be predicted;
arranging a candidate stem structure based on a free energy of each base constituting said stem structure;
arranging a defined stem structure from said candidate stem structure;
investigating a sequence structure state of said RNA sequence based on the basic information of said defined stem structure;
calculating a sequence energy state of each base constituting said RNA sequence based on said sequence structure state; and
arranging a candidate additional stem structure as a new defined stem structure based on a sequence energy state of the secondary structure of said RNA sequence as reflected with said defined stem structure and on a sequence energy state of a new secondary structure as reflected on said secondary structure with the candidate additional stem structure selected from said candidate stem structure.

## Description

### Field of Invention

The present invention relates to a method for predicting secondary structure of RNA, an apparatus for predicting using the method for predicting, and a predicting program carrying out the method for predicting.

### Related art

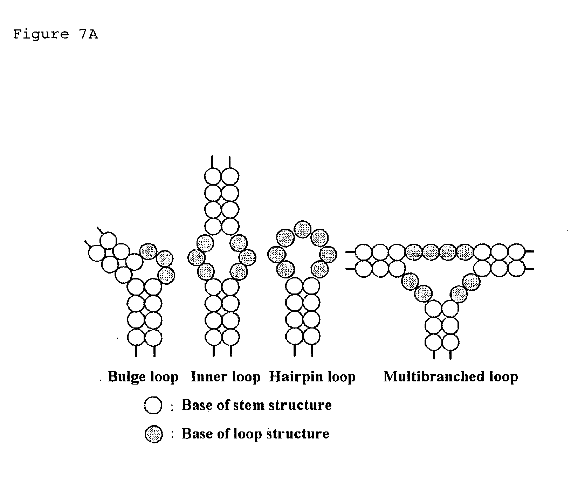

RNA is a nucleic acid consisting of 4 type of bases including adenine (A), cytosine (C), guanine (G) and uracil (U), and hydrogen bonds between A, and U and G and C is formed in RNA to form a base pair, thereby forming various type of secondary structure in accordance with its combination. The type of the secondary structure of RNA includes a stem structure which is a region comprising continuous base pairs, and the various secondary structures as shown in for example in Figure 7A. Especially, in the functional RNA, the higher-order structure including secondary structure is intimately involved in the function of RNA. So, it is very important to know the structure of RNA. However, a large amount of labor, cost and the others is necessary to experimentally analyze the RNA structure. Therefore, the method carrying out the simulation of structural prediction using the computer has been investigated. An example of the method for predicting of the secondary structure in the prior art includes, for example, Patent-related document 1.

Among the method for predicting the secondary structure of RNA in the prior art, there are two methods as the method for predicting the secondary structure from one RNA sequence. One of the two methods is to calculate the free energy using the dynamic programming, and the other is a method in which a candidate stem structure is primary listed and the combination thereof is optimized. These methods are described in Non-patent-related document 1. Especially, Non-patent-related document 2 describes in detail with regard to the prediction of the secondary structure with the dynamic programming and parameters used in the calculation of the free energy.

In case of the method for predicting the secondary structure with the dynamic programming, although the calculation is relatively fast, the prediction of pseudonot structure is difficult. On the other hand, in the method for optimizing the combination, although the pseudonot structure can be predicted, the calculation is relatively slow.

In addition, even in case of using the above-mentioned methods, there is a problem that cannot use any parameters of pseudonot structure for predicting its structure, since the value of the free energy at forming the pseudonot structure in RNA is not experimentally investigated.

Further, although there is a predicting method of the secondary structure from the evolutional relationship of a plurality of sequence for predicting the secondary structure of RNA (the method using the sequence alignment), the method cannot be used for prediction of the RNA structure which is artificially synthesized, due to its nature.
Patent-related document 1
   Japanese Patent Application Publication No. 154677/1996
Non-patent-related document 1
   Minoru Kanehisa, "Invitation to post genome information, Kyoritsu Shuppan Co. Ltd., June, 10, 2001, p. 108-111 Non-patent-related document 2
   Translation supervised by Yasushi Okazaki and Hidemasa Bounou, "Bioinformatics: Sequence and Genome Analysis", Medical Sciences International Ltd., p.212-242 Non-patent-related document 3
   Gorodkin et al., "Discovering common stem-loop motifs in unaligned RNA sequences", 2001, Nucleic Acids Research, vol. 29. no. 10, p. 2135-2144

### Disclosure of Invention

### Problem to be solved in the present invention

The present invention is made in accordance with the above-mentioned problems. The present invention is to provide a method for predicting secondary structure of RNA capable of predicting the secondary structure which has been difficult to predict the secondary structure including pseudonot structure, and an apparatus for predicting secondary structure of RNA using the method for predicting.

### Means for solving the problem

The method for predicting secondary structure of RNA according to the present invention is characterized in that:
A method for predicting secondary structure of RNA comprising the steps of:
   searching base capable of forming a stem structure from the RNA sequence to be predicted;
   arranging a candidate stem structure based on a free energy of each base constituting said stem structure;
   arranging a defined stem structure from said candidate stem structure;
   investigating a sequence structure state of said RNA sequence based on the basic information of said defined stem structure;
   calculating a sequence energy state of each base constituting said RNA sequence based on said sequence structure state; and
   arranging a candidate additional stem structure as a new defined stem structure based on a sequence energy state of the secondary structure of said RNA sequence as reflected with said defined stem structure and on a sequence energy state of a new secondary structure as reflected on said secondary structure with the candidate additional stem structure selected from said candidate stem structure.

The apparatus for predicting secondary structure of RNA according to the present invention is characterized in that:
An apparatus for predicting secondary structure of RNA comprising:
   means for searching candidate stem structure, arranging a candidate stem structure by searching a base which can form a stem structure among the RNA sequence to be subjected;
   means for arranging defined stem structure, arranging a defined stem structure from said candidate stem structure;
   means for investigating sequence structure state, investigating a sequence structure state of said RNA sequence based on the basic information of said defined stem structure;
   means for calculating sequence energy state, calculating a sequence energy state of each base constituting said RNA sequence based on said sequence structure state; and
   means for searching additional stem structure, arranging a candidate additional stem structure as a new defined stem structure based on a sequence energy state of the secondary structure of said RNA sequence as reflected with said defined stem structure and on a sequence energy state of a new secondary structure as reflected on said secondary structure with the candidate additional stem structure selected from said candidate stem structure.

The predicting program for secondary structure RNA according to the present invention is characterized in that:
A predicting program for secondary structure RNA carrying out the steps of:
   searching base capable of forming a stem structure from the RNA sequence to be predicted;
   arranging a candidate stem structure based on a free energy of each base constituting said stem structure;
   arranging a defined stem structure from said candidate stem structure;
   investigating a sequence structure state of said RNA sequence based on the basic information of said defined stem structure;
   calculating a sequence energy state of each base constituting said RNA sequence based on said sequence structure state; and
   arranging a candidate additional stem structure as a new defined stem structure based on a sequence energy state of the secondary structure of said RNA sequence as reflected with said defined stem structure and on a sequence energy state of a new secondary structure as reflected on said secondary structure with the candidate additional stem structure selected from said candidate stem structure.

### Effect of Invention

The first effect of the present invention is capable of predicting the secondary structure comprising pseudonot structure with the calculation of the free energy.

The reason is that the pseudonot structure is replaced with the other combination of the secondary structure in accordance with the patter of the structure around the stem structure to predict its structure. Brief explanation of Drawing

Figure 1 is a schematic diagram showing an example of apparatus for predicting secondary structure of RNA according to the present invention.
Figure 2 is an example of flowchart method for predicting secondary structure of RNA according to the present invention.
Figure 3 is a flowchart of searching candidate stem structure.
Figure 4 is a flowchart of investigating sequence structure state.
Figure 5 is a flowchart of calculating sequence energy state.
Figure 6 is a flowchart of searching additional stem structure.
Figure 7A is a schematic diagram showing secondary structure of RNA.
Figure 7B is an example of determination formula of secondary structure in the present invention.
Figure 8 is a schematic diagram showing the stem structure region.
Figure 9 is a schematic diagram showing an example of unretrieved region.
Figure 10 shows an example of the structure state of the input RNA sequence and the corresponding free energy.
Figure 11 is another schematic diagram showing an example of unretrieved region.
Figure 12 shows an example of the structure state of the input RNA sequence and the corresponding free energy.
Figure 13 is still another schematic diagram showing an example of unretrieved region.

### Explanation of Notation

- 1: input device
- 2: data processing device
- 3: storage device
- 4: output device
- 21: means for searching candidate stem structure

- 22: means for arranging defined stem structure
- 23: means for investigating sequence structure state
- 24: means for calculating sequence energy state
- 25: means for searching additional stem structure
- 26: means for calculating sequence structure energy state
- 31: defined value storage unit
- 32: candidate stem structure storage unit
- 33: defined stem structure storage unit
- 34: sequence structure state storage unit
- 35: sequence energy state storage unit

### Best mode for carrying out the present invention

The present invention is considered to be categorized in one of methods for optimizing a combination of the stem structure with one RNA. The prediction uses the calculation of free energy. The pseudonot structure which is related to calculate the free energy is treated and the other structural combination as already known in positional relationship to the circumference of the stem structure to achieve the calculation of the free energy.

Hereinafter, the preferred embodiment of the present invention will be explained with reference to the Drawing.

The apparatus for predicting secondary structure of RNA according to the present invention comprises an input device 1 such as keyboard, a data processing device (computer; central processing unit; processor) 2 operated by the program control, a storage device 3 storing the information, and a output device 4 such as the display device and printing device.

The storage device 3 comprises a defined value storage unit 31, a candidate stem structure storage unit 32, a defined stem structure storage unit 33 and a sequence structure state storage unit34 and sequence energy state storage unit 35.

The defined value storage unit 31 preliminary stores numerical information which is changed in the calculation, including value of free energy due to the continuous base pair, vale of free energy due to forming the loop structure, permissible minimum length of the stem structure, length of pseudonot structure, number of trial for prediction of secondary structure.

The candidate stem structure storage unit 32 stores various information related to the candidate stem structure which is a candidate portion of the stem structure and is searched by the means for searching candidate stem structure 21. For example, the candidate stem structure storage unit 32 stores: a base constituting the candidate stem structure; an information in what number of bases the base is located from the end of the RNA sequence in the RNA sequence as input (hereinafter, also referred to as an input RNA sequence); the value of the free energy at which the candidate stem structure forms the stem structure; and the others. In such a case, the candidate stem structure may be listed in accordance with the free energy in ascending order possessed in each stem structure, or in accordance with the order as desired by the user.

The defined stem structure storage unit 33 stores where the candidate stem structure which is determined to select at the cycle is stored in the candidate stem structure storage unit 32.

The sequence structure state storage unit 34 stores the result as determined by the means for investigating sequence structure state 23, including what structure state is constituted by each bases in the process of the calculation with regard to the input RNA sequence. Example of the structure state includes a portion of stem, a portion of bulge loop, a portion of inner loop, a portion of hairpin loop, a portion of multibranched loop, single strand, end structures such as a portion of one end of RNA sequence.

The sequence energy state storage unit 35 stores the result value in each base (for example, matter indicating the energy state in each structure state) as calculated by the means for calculating sequence energy state 24 based on the free energy in each structure state as stored in the sequence structure state storage unit 34. Each adjacent base contained in the same structure possesses the identical value each other. For example, all of bases constituting the same portion of the inner loop possesses the value of the free energy possessing its inner loop.

The data processing device 2 comprises means for searching candidate stem structure 21, means for arranging defined stem structure 22, means for investigating sequence structure state 23, means for calculating sequence energy state 24 and means for searching additional stem structure 25.

The means for searching candidate stem structure 21 searches a region in which the stem structure can be formed, among the input RNA sequence as input from the input device 1, using the information stored in the defined value storage unit 31 (e.g. value of free energy due to the continuous base pair, vale of free energy due to forming the loop structure, permissible minimum length of the stem structure, length of pseudonot structure, number of trial for prediction of secondary structure), and calculates the free energy possessed in case of the stem structure being formed. The means for searching candidate stem structure 21 arranges the region in which the stem structure can be formed as obtained from the searching and the calculation, as the candidate stem structure, and stores the candidate stem structure into the candidate stem structure storage unit 32 and the free energy of each candidate stem structure into the candidate stem structure storage unit 32 as the result of searching.

The means for arranging defined stem structure 22 receives the information of the candidate stem structure (e.g. the information of the base, the information of the free energy) from the candidate stem structure storage unit 32, selects the candidate stem structure to be investigated, calculated and searched as performed later, and stores it into the defined stem structure storage unit 33. The candidate stem structure to be selected differs in accordance with the searching, investigating, calculating and the other with regard to the input RNA sequence. For example, when the secondary structure prediction is at the first round, the candidate stem structure is searched with regard to the RNA sequence input from the input device 1, and these candidate stem structures are listed as mentioned above. Then, the candidate stem structure which is initially selected by the means for arranging defined stem structure 22 is the candidate stem structure listed at the top thereof by the means for searching candidate stem structure 21. In such case, the means for arranging defined stem structure 22 stores this candidate stem structure into the defined stem structure storage unit 33 as the defined stem structure. In addition, when the secondary structure prediction is the second round, the means for arranging defined stem structure 22 arranges the next candidate stem structure of the candidate stem structure which is selected by the means for arranging defined stem structure 22 at the first round (that is, the top of the candidate stem structure in that list as stored in the defined stem structure storage unit 33), as the defined stem structure. In such a manner, the means for arranging defined stem structure 22 arranges the listed candidate stem structure as the defined stem structure at there order in accordance with the round of the secondary structure prediction.

The means for investigating sequence structure state 23 receives various information stored in the defined stem structure storage unit 33, such as the basic information of the defined stem structure, and assigns the corresponding base in the input RNA sequence as being in a condition of containing a part of the stem structure. Next, the means for investigating sequence structure state 23 divides the input RNA sequence into regions of constituting the stem structure and the other bases at the end base constituting this stem structure. Then, the means for investigating sequence structure state 23 determines the structure state in positional relationship between each region as divided and the stem structure, and stores the result into the sequence structure state storage unit 34.

The means for calculating sequence energy state 24 receives the information with regard to the free energy possessed in the base pair and the loop structure wherein the free energy is experimentally investigated, from the defined value storage unit 31, and receives the information of the structure state of the input RNA sequence from the sequence structure state storage unit 34. Then, the means for calculating sequence energy state 24 sequentially calculates a value of free energy corresponding to the structure state of each region of the input RNA sequence, and makes each base contained in the region to hold the value, and stores the result into the sequence energy state storage unit 35.

The means for searching additional stem structure 25 receives the information of candidate stem structure from the candidate stem structure storage unit 32, and sets the candidate stem structure which is only constituted by the base not overlapped with each base contained in the stem structure stored in the defined stem structure storage unit 33 as a candidate of stem structure as added (hereinafter, also referred to as candidate additional stem structure).

Next, the means for searching additional stem structure 25 searches as to whether the candidate additional stem structure is set as the defined stem structure. That is, the means for searching additional stem structure 25 compares the structure state of the input RNA sequence in which the defined stem structure stored in the defined stem structure storage unit 33 is reflected, with the structure stat of the RNA sequence in which the candidate additional stem structure is reflected in the input RNA sequence as reflected in the defined stem structure, in view of the free energy, and determines the candidate additional stem structure with which the structure state with lower free energy can become, as the defined stem structure as stem structure to be added.

It is explained as to determining the means for searching additional stem structure 25 as the defined stem structure as the stem structure to be added. The means for searching additional stem structure 25 receives the information of the structure at each base of the secondary structure formed with the defined stem structure stored in the defined stem structure storage unit 33, and receives the energy state of each structure containing each base in the secondary structure, from the sequence energy state storage unit 35. Next, the means for searching additional stem structure 25 calculates an amount of change (a difference) between the free energy of this secondary structure and the free energy of the whole input RNA sequence due to the change of the secondary structure as created by actually adding the candidate additional stem structure in this secondary structure.

The calculation of the amount of change is performed with regard to all of the candidate additional stem structures. The candidate additional stem structure which gives a negative minimum value among the amount of change is determined as the stem structure to be added, and stored in the defined stem structure storage unit 33 as the defined stem structure. The defined stem structure is reflected into the input RNA sequence to provide a certain secondary structure. With regard to the reflected secondary structure, the means for investigating sequence structure state 23 calculates a sequence structure state, and the means for calculating sequence energy state 24 calculates the free energy thereof.

On the other hand, when the minimum value of the amount of change is positive, the secondary structure prediction at its round is terminated at that time, and the stem structure stored in the defined stem structure storage unit 33 at that time is output in just proportion to the output device 4. When the round of the secondary structure prediction at that time is less than the predetermined round of the secondary structure prediction stored in the defined value storage unit 31, subsequent steps of the step using the means for arranging defined stem structure22 are repeated. Then, when the predetermined round is achieved, the calculation is terminated.

In the present invention, the input device 1, the data processing device 2, the storage device 3 and the output device 4 may be provided in the integrated computer, and may be provided in different computers through a line such as the Internet.

It should be noted that, among the arrows between the data processing device 2 and the storage device 3, arrows from each means of the data processing device 2 is indicated as dashed arrows, and arrows from each unit of the storage device 3 is indicated as solid lines.

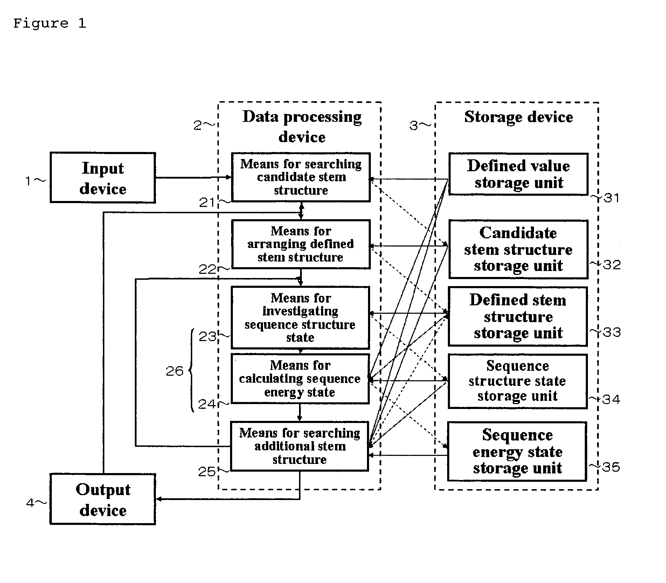

Next, the present invention will be explained in detail with reference to Figures 1, and 2 to 6.

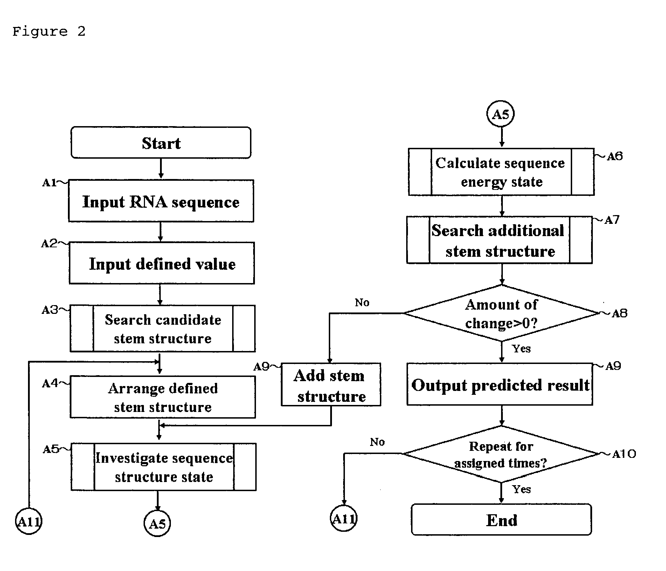

The character string information of the RNA sequence given from the input device 1 (input RNA sequence) is supplied to the means for searching candidate stem structure 21 (step A1 of Figure 2). The information of the defined value such as value of free energy due to the continuous base pair, vale of free energy due to forming the loop structure, permissible minimum length of the stem structure, length of pseudonot structure, number of trial for prediction of secondary structure is preliminary stored in the defined value storage unit 31. In case of changing these values, it given from the input device 1 as the same as the sequence information (step A2 of Figure 2), and it is stored in the defined value storage unit 31.

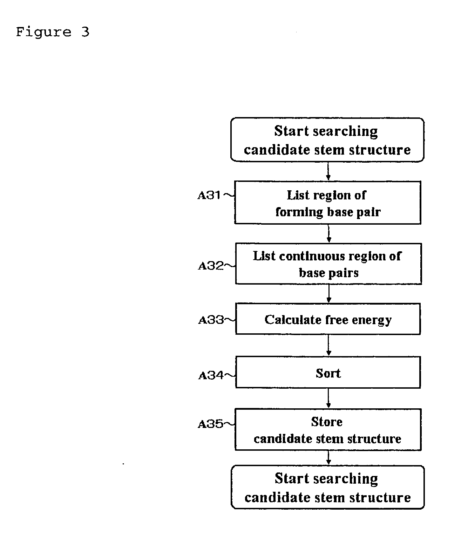

The means for searching candidate stem structure 21 searches a possible region forming the base pair from each base constituting the input RNA sequence (step A31 of Figure 3), and searches a possible portion forming the stem structure (portion of continuous base pairs) (step A32), based on the information of the possible region. After the summation of the free energy of the structure due to the continuous base pairs is calculated (step A33 of Figure 3, candidates of the searched stem structure is sorted (aligned) in ascending order of the free energy (step A34). The means for searching candidate stem structure 21 sets the information of the base constituting each candidate stem structure and the information of free energy of the candidate stem structure, and stores it into the candidate stem structure storage unit 32 (step A35). The stored candidate stem structure is picked up from the top thereof in accordance with a round (trial round) of the secondary structure prediction, and is stored in the defined stem structure storage unit 33 as the first stem structure as determined to form the stem structure.

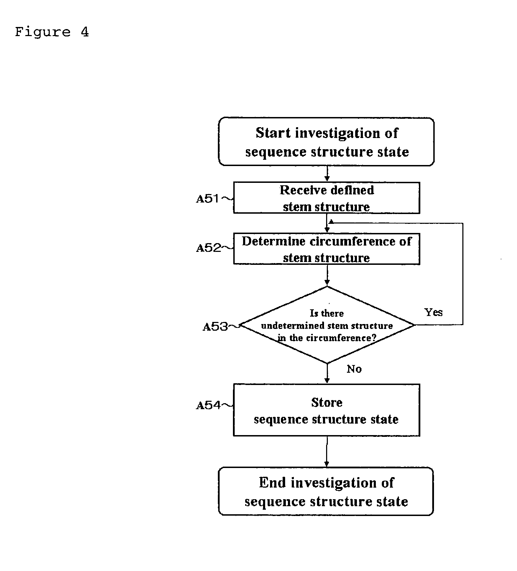

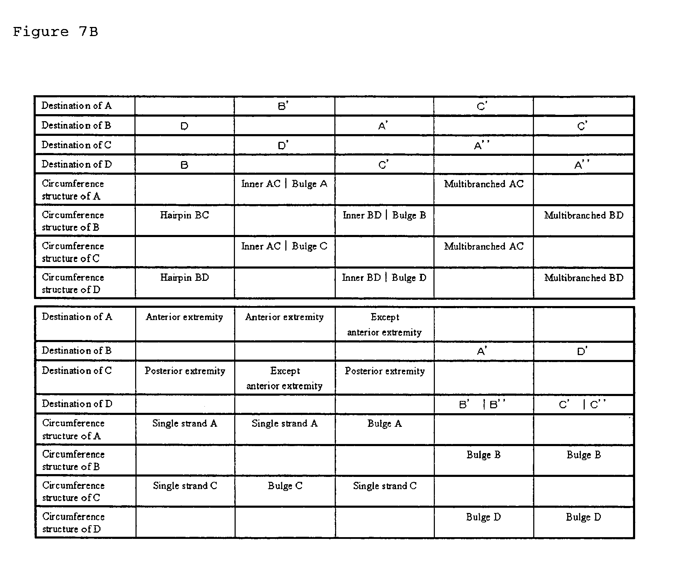

The means for investigating sequence structure state 23 which received the information of the defined stem structure from defined stem structure storage unit 33 lays out the defined stem structure on the input RNA sequence (step A51 of Figure 4). After laying out, with regard to the region of the base not belonging to the stem structure, the secondary structure of each region is searched (determined) in accordance with the positional relationship of the neighborhood stem structure (step A51 of Figure 4). This search is performed by making it to belong to the well-known structure in the secondary structure of the RNA sequence. Figure 7A shows a schematic diagram showing secondary structure of RNA, and Figure 7B shows an example of determination formula of secondary structure in the present invention.

Here, in Figure 7B:
a base which is contained in the stem structure and which is most proximity to the beginning of the RNA sequence is assigned as a standard of mark "A";
a base which is located at opposite end of the same stem structure containing the standard is assigned as mark "B";
a base forming the base pair with the standard of "A" is assigned as mark "C";
a base forming the base pair with "B" is assigned as mark "D".
In addition, whether the base is contained in the same stem structure is distinguished with the presence or absence of statement "'" or "''".
In addition, in case of absence of the combinations of "(A,C)" or "(B,D)" in the Table, the circumference structure thereof is assigned as the bulge loop.

It should be noted that it can be considered that the base corresponding to the end of the stem structure does not form the loop structure. However, in the investigation, it deems the base to form the unique secondary structure. By doing so, the circumference structure of a stem structure is investigated. When there is an uninvestigated region in the circumference of the defined stem structure, the investigation of the circumference structure is performed. When there is not an uninvestigated (undetermined) region, the structure state as investigated is stored in the sequence structure state storage unit 34 (steps A53 and A54 of Figure 4).

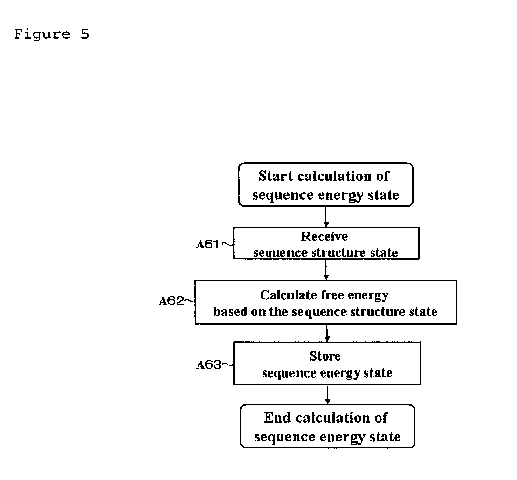

After the structure state of the sequence is determined, the means for calculating sequence energy state 24 receives the structure state of the sequence from the sequence structure state storage unit 34 (step A61 of Figure 5), and calculates the free energy of each region, using the value of the free energy at forming the loop structure stored in the defined value storage unit 31 as the defined value. In such a case, all of the bases contained in the region may possess the same value (step A62 of Figure 5). The energy state of each base of the sequence is stored in the sequence energy state storage unit 35 (step A63 of Figure 5).

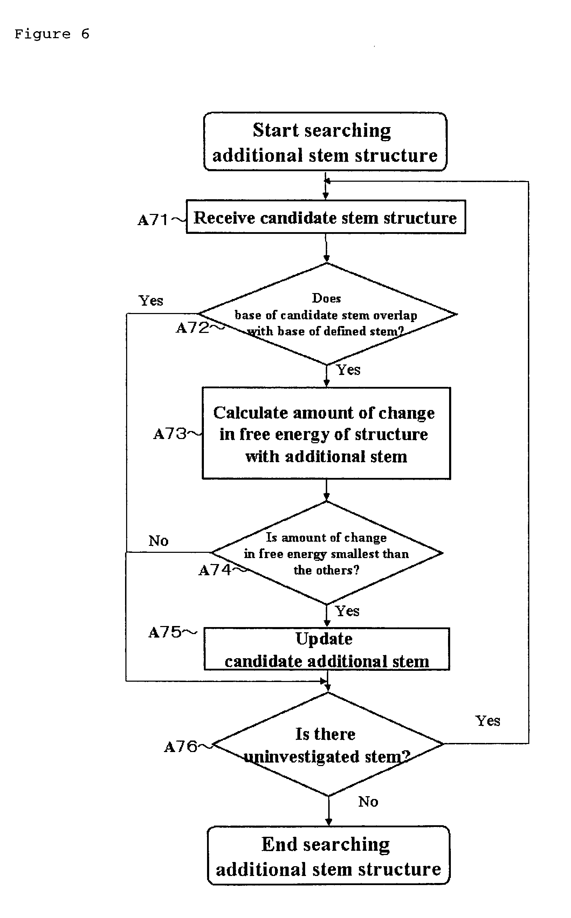

After the energy state of the sequence is obtained, the means for searching additional stem structure 25 receives the candidate stem structure from the candidate stem structure storage unit32 (step A71 of Figure 6), and investigates as to whether the base constituting the candidate stem structure is overlapped with the base of the defined stem structure (step A72 of Figure 6). When there is an overlap, the investigation of the overlap with regard to the next candidate stem structure is performed. When there is not an overlap, this candidate stem structure is assigned as a candidate of the structure (candidate additional stem structure) to be added as the defined stem structure. The means for searching additional stem structure 25 calculates the amount of change of the free energy originated from each structure state between a structure state obtained by reflecting the defined stem structure on the input RNA sequence and a structure state obtained by reflecting the candidate additional stem structure on this structure state (step A73 of Figure 6). Minimum value (largest value in the negative direction) of the amount of change estimated at this time and the information of the candidate stem structure at which the value is estimated are temporarily stored, and the minimum amount of change and the candidate of the additional stem structure are rewritten at each time when the minimum value is renewed (steps A74 and A75 of Figure 6).

The subsequence steps of the investigation of the overlap are repeated until there is not uninvestigated candidate stem structure (step A76 of Figure 6).

After there is not uninvestigated candidate stem structure, the means for searching additional stem structure 25 determines as to whether the value held as the minimum amount of change at that time is positive or negative (step A8 of Figure 2).

When the amount of change is negative, the candidate additional stem structure held in the means for searching additional stem structure 25 at that time is added to the defined stem structure storage unit 33 as the defined stem structure, and the information in the defined stem structure storage unit 33 is renewed (step A9 of Figure 2). Then, the subsequent steps (steps A5 to A9) of the step using the means for investigating sequence structure state 23 are repeated again.

When the amount of change is positive, the candidate additional stem structure held at that time is discarded. Each defined stem structure stored in the defined stem structure storage unit 33 at that time is a prediction result of the secondary structure for the input RNA sequence, and the result is output to the output device 4 (step A10 of Figure 2).

After the result is output, the trial round of the secondary structure prediction at present is determined (step A11 of Figure 2). When the trial round at present is less than the input trial round as the defined value, among the candidate stem structure stored in the candidate stem structure storage unit 32, the next sorted candidate stem structure of the candidate stem structure assigned in the means for arranging defined stem structure 22 at the round (the defined stem structure in case of the first round) is assigned as the defined stem structure, and the subsequent steps of the step using means for investigating sequence structure state 23 are repeated (step A4 of Figure 2). After the predetermined trial round is achieved, the calculation is finished.

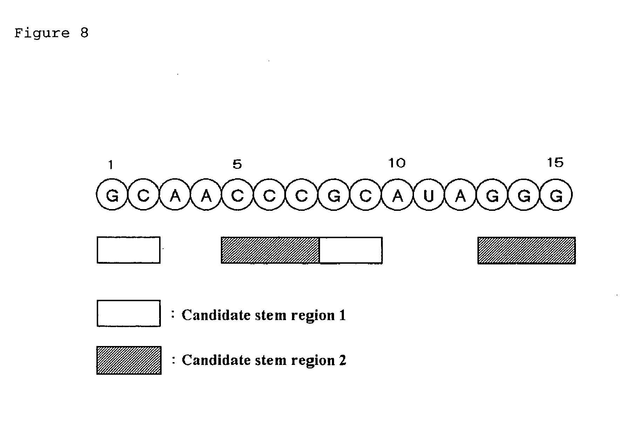

Next, the operation of the present embodiment will be explained using specific examples with reference to Figures 8 to 13 and the others.

It is supposed that GCAACCCGCAUAGGG is given in the input device 1 as the input RNA sequence. If any defined values are not input at that time, the information as primary input in the defined value storage unit 31 such as free energy is used for the following calculation. It should be noted that, as a matter of convenience, the base "G" corresponding to numeral "1" as stated in Figure 8 refers to as 5' end, and the base "G" corresponding to numeral "15" as stated in the Figure refers to as 3' end.

The means for searching candidate stem structure 21 finds and lists continuous portion of base pairs of G-C, A-U and G-U such as white area (candidate stem region 1) and shaded area (candidate stem area 2) of Figure 8 as the candidate stem structure. The free energy of the candidate stem region is estimated as the summation of the unique value mainly depending on the type of alignment of the base pair. Accordingly, if the value of the free energy in case of continuous base pair of G-C is supported as -2, the free energy of the candidate stem region is - 4, and free energy of the candidate stem region 2 is -6. The means for searching candidate stem structure 21 sorts each candidate stem structure in ascending order of free energy, and stores it in the candidate stem structure storage unit 32. In case of the input RNA sequence as shown in Figure 8, the means for searching candidate stem structure 21 stores the order of each candidate stem region (candidate stem region 2 and candidate stem region 1) sorted as mentioned, the base constituting these candidate stem region and the value of the free energy of the region in the candidate stem structure storage unit 32.

Next, the means for arranging defined stem structure 22 arranges the candidate stem region as listed in the top of the list of candidate stem structures stored in the candidate stem structure storage unit 32 as the first defined stem structure, and stores it in the defined stem structure storage unit 33.

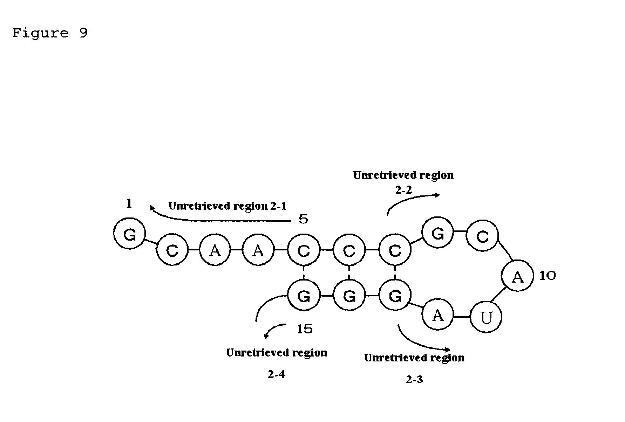

The means for investigating sequence structure state 23 initially receives the information of the candidate stem region 2 among the defined stem structure stored in the defined stem structure storage unit 33, and assigns as being in a condition that a base corresponding the input RNA sequence is contained in the part of the candidate stem region 2. If a stem structure is determined, there can be 4 undetermined structure regions around the stem structure. That is, the 4 undetermined structure regions are, as shown in Figure 9, a region which is from 5^{th} residue of 5' end to the 5' end direction of the input RNA sequence (an unretrieved region 2-1), a region which is from 7^{th} residue of 5' end to the 3' end direction of the input RNA sequence (an unretrieved region 2-2), a region which is from 7^{th} residue of 5' end to 3' end of the input RNA sequence (unretrieved region 2-3) and a region which is from 15^{th} residue of 5' end to 3' end direction of the input RNA sequence (an unretrieved region 2-4). Each region is a region which is from the region of the original stem structure as a starting point to the region of the other stem structure or to the end of the sequence.

The means for investigating sequence structure state 23 initially investigates the proximal region to 5' end of the input RNA sequence (in this case, the unretrieved region 2-1). So, in this case, there is no stem structure in the region from 5^{th} residue to 5' end. Accordingly, it is found that the unretrieved region 2-1 is connected to 5' end of the input RNA sequence. In this case, the unretrieved region 2-1 is assigned as a single strand region comprising 4 bases. Next, the unretrieved region 2-2 and the unretrieved region 2-3 are searched. So, it is found that there regions are connected to an anterior extremities of the unretrieved region 2-3 and the unretrieved region 2-2, respectively. In this case, it is found that the unretrieved region 2-2 (or the unretrieved region 2-3) forms the hairpin loop structure comprising 5 bases. Finally, the searching of the unretrieved region 2-4 is performed. It is found that the unretrieved region 2-4 is connected to the end of the sequence, and there is no base in the region. So, the determination of the circumference of stem structure with regard to the candidate stem region 2 is finished (step A52 of Figure 4).

In the secondary structure prediction of the input RNA sequence as shown in Figure 8, the defined stem structure stored in the defined stem structure storage unit 33 at this time is only the candidate stem region 2. Accordingly the investigation is finished.

After the searching is finished, the means for investigating sequence structure state 23 stores the information of the structure state of the investigated RNA sequence in the sequence structure state storage unit 34.

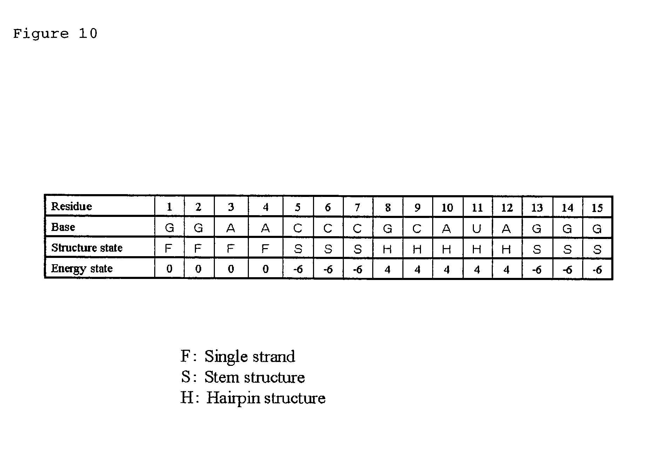

Next, the means for calculating sequence energy state 24 receives the information of the structure state from the sequence structure state storage unit 34, and calculates the free energy corresponding to each structure using the date of the free energy received from the defined value storage unit 31. In accordance with the information of the structure state, it is found that the input RNA sequence is constituted from the single strand region (corresponding to the unretrieved region 2-1) comprising 4 bases, the hairpin loop structure (corresponding to the unretrieved regions 2-2 and 2-3) comprising 5 bases, and the stem structure region comprising 3 G-C pairs. Accordingly, if the free energy of the single strand region is 0, and the free energy of the hairpin loop structure comprising 5 bases is 4, the means for calculating sequence energy state 24 stores the energy corresponding to each structure state in each bases in the sequence energy state storage unit35, as shown in Figure 10.

Next, the means for searching additional stem structure 25 receives the candidate stem structure only comprising the base not contained in the defined stem structure from the candidate stem structure storage unit 32 in the sorted order among the candidate stem structure stored in the candidate stem structure storage unit 32. In this case, the means for searching additional stem structure 25 receives the candidate stem region 1 as shown in Figure 8. In according to the information of the structure of input RNA sequence stores in the sequence structure state storage unit 34, the base constituting the candidate stem region 1 is not overlapped with the base contained in the current stem structure (i.e. the candidate stem region 2). Accordingly, the candidate stem region 1 is assigned as the candidate additional stem structure. Next, the candidate stem region 1 is added in the stem structure stored in the defined stem structure storage unit 33, and supplied to the means for investigating sequence structure state 23.

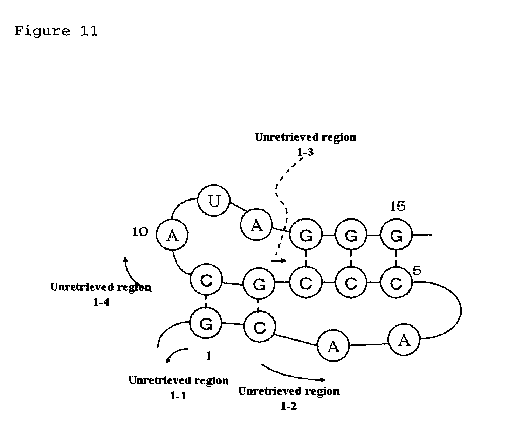

Next, the means for investigating sequence structure state 23 investigates the structure state in which the candidate stem region 1 is reflected on the structure stet of the input RNA sequence as shown in Figure 9, that is, the structure state of the input RNA sequence as shown Figure 11. That is, the means for investigating sequence structure state 23 which receives the candidate stem region 1 from the candidate stem structure storage unit 32 as mentioned above initially arranges the corresponding base of the input RNA sequence as being in a condition that the base is contained in part of the candidate stem region 1, as similar to the investigation for the candidate stem region 2. After that, the means for investigating sequence structure state 23 the structure state around the candidate stem region 1. That is, the means for investigating sequence structure state 23 searches a region which is from 1^{st} residue to 5' end direction (an unretrieved region 1-1), a region which is from 2^{nd} residue to 3' end direction (an unretrieved region 1-2), a region which is from 8^{th} residue to 5' end direction (an unretrieved region 1-2) and a region which is from 9^{th} residue to 3' end direction (an unretrieved region 1-4), respectively. First, with regard to the unretrieved region 1-1 and the unretrieved region 1-4, the unretrieved region 1-1 does not contain any bases since the region is just connected to the end of the sequence, while the unretrieved region 1-4 is connected to the other candidate stem region (in this case, the above-mentioned candidate stem region 2). In this case, the unretrieved region 1-1 is determined as the single strand region comprising 0 base, and the unretrieved region 1-4 is determined as the bulge loop structure comprising 3 bases. Next, the unretrieved region 1-2 and the unretrieved region 1-3 searched. These regions are connected to the same side of the chain in the same stem structure. In this case, the unretrieved region 1-2 and the unretrieved region 1-3 are determined as forming the bulge loop structure comprising 2 bases, and the bulge loop structure comprising 0 base. The information of the circumference structure state of the candidate stem region 1 at this time is sent to the means for calculating sequence energy state 24.

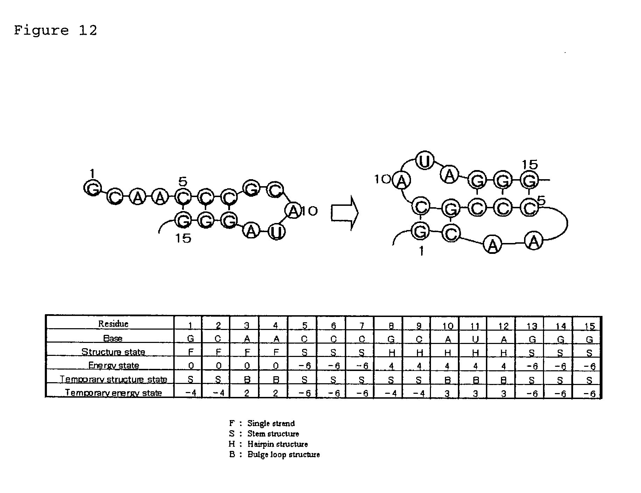

The means for calculating sequence energy state 24 at this time calculates the free energy using the structure information around the candidate stem region 1 previously determined as mentioned above. If the free energy of the bulge loop structure comprising 2 bases is 2, and the free energy of the bulge loop structure comprising 3 bases is 3, the free energy is calculated as shown in Figure 12. Here, in comparison of Figure 9 of the original structure and Figure 11 of the structure obtained by reflecting the candidate stem region 1, what portion of the structure is changed by forming the candidate stem region 1 is the single strand region which is from 5^{th} residue to 5' end direction, and the region of hairpin loop structure which is from 7^{th} residue to 13^{th} residue. It is found that the stem structure of the candidate stem region 1, the bulge loop structure which is from 2^{nd} residue to 5^{th} residue, and the bulge loop structure which is from g^{th} residue to 13^{th} residue is newly formed in the structure as shown in Figure 11, instead of the structure of the region. The local free energy in this case is changed from 4 which is summation of the free energies originated from the single strand region and the hairpin loop structure as shown in Figure 9, to 1 which is summation of the free energies originated from the stem structure of the candidate stem region 1 and 2 bulge loop structures. This is that the amount of change in the free energy is negative. Accordingly, the candidate stem region 1 is accepted as the additional stem structure (step A74 and step A75). The candidate stem region 1 is stored in the defined stem structure storage unit 33 as a new defined stem structure, since the other defined stem structure than the candidate stem region 1 is not stored in the candidate stem structure storage unit (step A76).

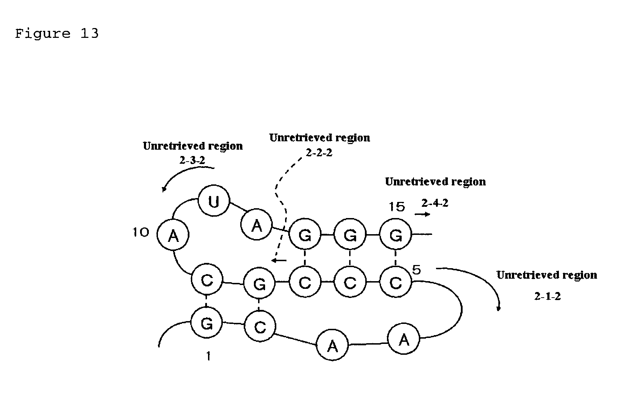

Next, the means for investigating sequence structure state 23 investigates again the whole structure state of the input RNA sequence in response to increasing the defined stem structure. The investigation of the structure is performed in the circumference structure in ascending order of the distance from the anterior proximity of the sequence to the anterior proximity base among the bases forming each stem structure. In this case, the candidate stem region 1 and the candidate stem region 2 as shown in Figure 8 are investigated in its order. With regard to the determination of the circumference structure of the candidate stem region 1, it is the same as mentioned above. With regard to the circumference structure of the candidate stem region 2, there is a region which is from 5^{th} residue to 5' end direction (an unretrieved region 2-1-2), a region which is from 7^{th} residue to 3' end direction (an unretrieved region 2-2-2), a region which is from 13^{th} residue to 5' end direction (an unretrieved region 2-3-2) and a region which is from 15^{th} residue to 3' end direction (an unretrieved region 2-4-2), as referred to Figure 13. In addition, with regard to the unretrieved region 2-1-2 and the unretrieved region 2-4-2, it is found that the unretrieved region 2-1-2 is connected to the stem structure, and the unretrieved region 2-4-2 is connected to the end of the sequence. Therefore, the unretrieved region 2-1-2 is determined as the bulge loop structure comprising 2 bases, and the unretrieved region 2-4-2 is determined as the single strand region comprising 0 base. In addition, in the unretrieved region 2-2-2 and the unretrieved region 2-3-2, it is found that it is connected to the same side of the chain in the same stem structure. Accordingly, it is found that the unretrieved region 2-2-2 and the unretrieved region 2-3-2 is bulge loop structure comprising 0 base and the bulge loop structure comprising 3 bases, respectively. Here, the unretrieved region 2-1-2, the unretrieved region 2-2-2 and the unretrieved region 2-3-2 are the region which is already determined as the circumference structure of the candidate stem region 1, and this determination is not incompatible to the result obtained from the determination of the circumference structure of the candidate stem region 2. Accordingly, the result of the determination for the circumference structure of the candidate stem region 2 is used without change. So, since all of the circumference structure of the defined stem structure at present is determined, the means for investigating sequence structure state 23 stores the information of the structure state of the RNA sequence investigated as mentioned above in the sequence structure state storage unit 34 by overwriting the previous one.

The means for calculating sequence energy state 24 performs the same steps at calculating the free energy of the above-mentioned whole RNA sequence, and stores it in the sequence energy state storage unit 35 by overwriting the previous one.

Next, the means for searching additional stem structure 25 refers to the candidate stem structure to be added in accordance with the list of the candidate stem structures stored in the candidate stem structure storage unit 32. In this case, since the determination for all candidate stem structures to be as candidates in the sequence as shown in Figure 8 is finished, the means for searching additional stem structure 25 determines that there is no stem structure to be added (step A76).

The first of the secondary structure prediction with regard to the input RNA sequence is finished, and a structure wholly comprising the candidate stem region 1 and the candidate stem region 2 of the stem structure stored in the defined stem structure storage unit 33 is output by the output device 4, wherein the structure is stored in the sequence structure state storage unit 34 (step A10). Here, in case of 2 or more trial rounds of the secondary structure prediction stored in the defined value storage unit 31, the means for arranging defined stem structure 22 receives the candidate stem region 1 from the candidate stem structure storage unit 32 as the candidate stem structure, and the result obtained by performing the above-mentioned procedure is output.

As the other aspect of the present invention, the two steps using the means for investigating sequence structure state 23 and the means for calculating sequence energy state 24 as shown in Figure 2. That is, the step may be performed by using a means for calculating sequence structure energy state 26 in which the structure of the unretrieved region is determined in the means for investigating sequence structure state 23, the energy of the region is calculated, the information of the structure is stored in the sequence structure state storage unit 34, and the information of the energy is stored in the sequence energy state storage unit 35.

Therefore, the method for predicting secondary structure of RNA according to the present invention, the apparatus for predicting secondary structure of RNA according to the present invention and the predicting program for secondary structure RNA according to the present invention are a method for predicting performing the above-mentioned steps, a apparatus for predicting comprising each means performing the above-mentioned steps, and a predicting program carrying out the above-mentioned steps, respectively.

### (Example 1)

With regard to the following each sequence (sequences 1 to 22), the prediction of the secondary structure of RNA sequence was performed by using the method for predicting secondary structure of RNA according to the present invention, and the sensitivity and the specificity as disclosed in Non-patent-related document 3 was calculated. The result is shown in Table 1.

Sequence 1: GGAACCGGUGCGCAUAACCACCUCAGUGCGAGCAA
Sequence 2: GGAUCCCGACUGGCGAGAGCCAGGUAACGAAUGGAUCC
Sequence 3: GGACCGUCAGAGGACACGGUUAAAAAGUCCUCU
Sequence 4: GGCCGAAAUCCCGAAGUAGGCC
Sequence 5: GGCGAUACCAGCCGAAAGGCCCUUGGCAGCGUC
Sequence 6: CAUACUUGAAACUGUAAGGUUGGCGUAUG
Sequence 8: GGGAGCUUGAUCCCGGAAACGGUCGAUCGCUCCC
Sequence 9: GGCGAUACCAGCCGAAAGGCCCUUGGCAGCGUC
Sequence 11 GGAGAUCGCACUCCA
Sequence 12: CGAAACAUAGAUUCGA
Sequence 13: ACUUGGUUUAGGUAAUGAGU
Sequence 14: GGCGUGUAGGAUAUGCUUCGGCAGAAGGACACGCC
Sequence 17: GGACUGGGCGAGAAGUUUAGUCC
Sequence 20: GGAUCCCGACUGGCGAGAGCCAGGUAACGAAUGGAUCC
Sequence 21: GGGAAGGGAAGAAACUGCGGCUUCGGCCGGCUUCCC
Sequence 22: GGCACGAGGUUUAGCUACACUCGUGCC

### (Example 2)

With regard to the same sequences as mentioned in the Example 1, the sensitivity and the specificity was calculated except for performing the prediction of the secondary structure of the RNA sequence using MFOLD (http://www.bioinfo.rpi.edu/applications/mfold/old/rna/), in accordance with the Example 1. The result is shown in Table 2.

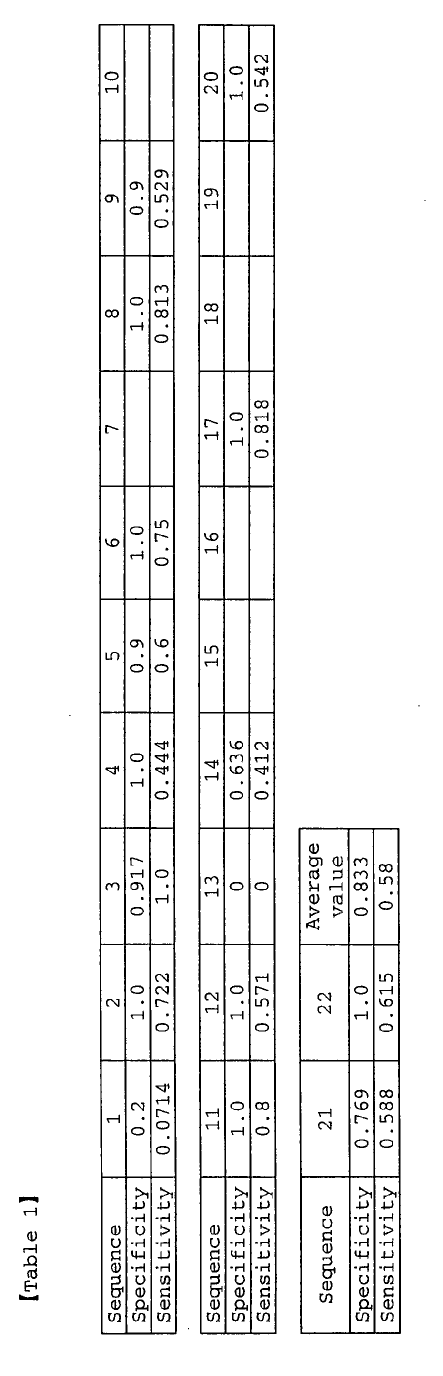

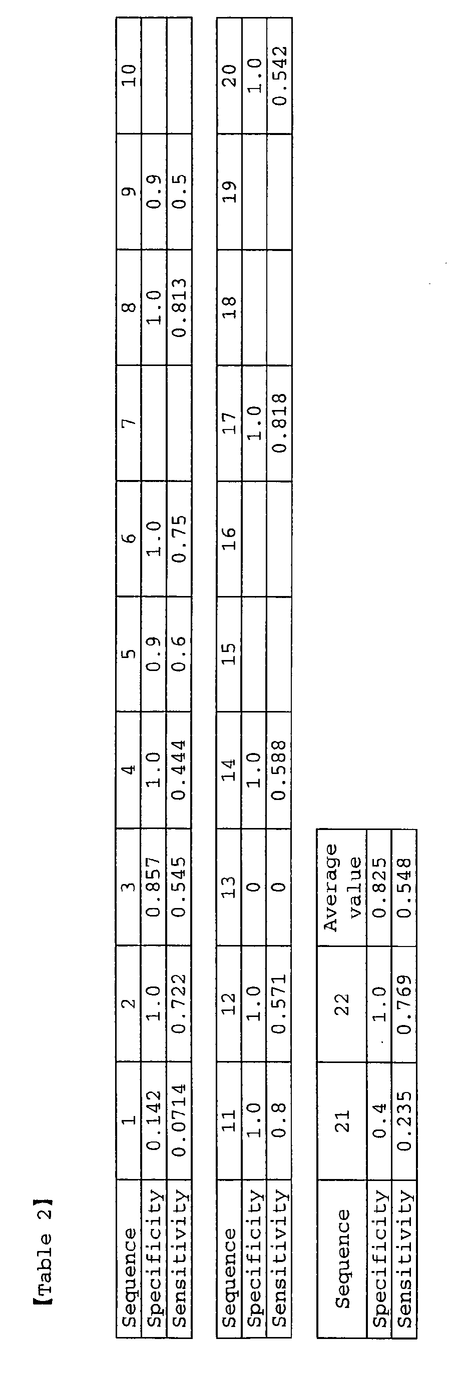

Generally, it is considered that the increase of the specificity and the sensitivity leads to improve the accuracy of the prediction. In comparison between the Example 1 and the Example 2 for the accuracy of the prediction of the method for predicting secondary structure of RNA according to the present invention, the average value was increased. Therefore, it is found that it is possible to predict the secondary structure of RNA by using the present invention with good accuracy.

With that, the present invention is explained with reference to the preferred embodiment of the present invention. Although it is explained by showing the certain example, it is obvious that any modifications and changes to the certain example can be made without departing from the wide sprit and the scope of the present invention as recited in the claims. That is, it should not be interpreted that the present invention is limited to the explanation of the certain example and the attached drawing.

## Claims

1. A method for predicting secondary structure of RNA comprising the steps of:
searching base capable of forming a stem structure from the RNA sequence to be predicted;
arranging a candidate stem structure based on a free energy of each base constituting said stem structure;
arranging a defined stem structure from said candidate stem structure;
investigating a sequence structure state of said RNA sequence based on the basic information of said defined stem structure;
calculating a sequence energy state of each base constituting said RNA sequence based on said sequence structure state; and
arranging a candidate additional stem structure as a new defined stem structure based on a sequence energy state of the secondary structure of said RNA sequence as reflected with said defined stem structure and on a sequence energy state of a new secondary structure as reflected on said secondary structure with the candidate additional stem structure selected from said candidate stem structure.

2. The method for predicting secondary structure of RNA according to claim 1, wherein said step of arranging the candidate stem structure is performed in ascending order of the free energy of the stem structure.

3. The method for predicting secondary structure of RNA according to claim 1 or 2, wherein said sequence structure state is a structure selected from the group consisting of the stem structure, the bulge loop structure, the inner loop structure, the hairpin loop structure, the multibranched loop structure, the single strand and the end structure of RNA sequence.

4. The method for predicting secondary structure of RNA according to any one of claims 1 to 3, wherein said step of calculating sequence energy state is a step of calculating the summation of the free energy of each base constituting said sequence structure state.

5. The method for predicting secondary structure of RNA according to any one of claims 1 to 4, wherein said step of arranging the candidate additional stem structure as a defined stem structure is a step of arranging the candidate additional stem structure as a new defined stem structure when an amount of change is negative, the amount of change being obtained by subtracting a sequence energy state of the secondary structure of said RNA sequence in which said defined stem structure is reflected on the secondary structure with a sequence energy state of new secondary structure in which the candidate additional stem structure selected from said candidate stem structure is reflected on the secondary structure.

6. An apparatus for predicting secondary structure of RNA comprising:
means for searching candidate stem structure, arranging a candidate stem structure by searching a base which can form a stem structure among the RNA sequence to be subjected;
means for arranging defined stem structure, arranging a defined stem structure from said candidate stem structure;
means for investigating sequence structure state, investigating a sequence structure state of said RNA sequence based on the basic information of said defined stem structure;
means for calculating sequence energy state, calculating a sequence energy state of each base constituting said RNA sequence based on said sequence structure state; and
means for searching additional stem structure, arranging a candidate additional stem structure as a new defined stem structure based on a sequence energy state of the secondary structure of said RNA sequence as reflected with said defined stem structure and on a sequence energy state of a new secondary structure as reflected on said secondary structure with the candidate additional stem structure selected from said candidate stem structure.

7. The apparatus for predicting secondary structure of RNA according to claim 6, wherein said means for searching candidate stem structure lists said candidate stem structure in ascending order of the free energy.

8. The apparatus for predicting secondary structure of RNA according to claim 6 or 7, wherein said sequence structure state is a structure selected from the group consisting of the stem structure, the bulge loop structure, the inner loop structure, the hairpin loop structure, the multibranched loop structure, the single strand and the end structure of RNA sequence.

9. The apparatus for predicting secondary structure of RNA according to any one of claims 6 to 8, wherein said means for calculating sequence energy state calculates the summation of the free energy of each base constituting said sequence structure state.

10. The apparatus for predicting secondary structure of RNA according to any one of claims 6 to 9, wherein said means for searching additional stem structure arranges the candidate additional stem structure as a new defined stem structure when an amount of change is negative, the amount of change being obtained by subtracting a sequence energy state of the secondary structure of said RNA sequence in which said defined stem structure is reflected on the secondary structure with a sequence energy state of new secondary structure in which the candidate additional stem structure selected from said candidate stem structure is reflected on the secondary structure.

11. A predicting program for secondary structure RNA carrying out the steps of:
searching base capable of forming a stem structure from the RNA sequence to be predicted;
arranging a candidate stem structure based on a free energy of each base constituting said stem structure;
arranging a defined stem structure from said candidate stem structure;
investigating a sequence structure state of said RNA sequence based on the basic information of said defined stem structure;
calculating a sequence energy state of each base constituting said RNA sequence based on said sequence structure state; and
arranging a candidate additional stem structure as a new defined stem structure based on a sequence energy state of the secondary structure of said RNA sequence as reflected with said defined stem structure and on a sequence energy state of a new secondary structure as reflected on said secondary structure with the candidate additional stem structure selected from said candidate stem structure.

12. The predicting program for secondary structure RNA according to claim 11, wherein said step of arranging the candidate stem structure is performed in ascending order of the free energy of the stem structure.

13. The predicting program for secondary structure RNA according to claim 11 or 12, wherein said sequence structure state is a structure selected from the group consisting of the stem structure, the bulge loop structure, the inner loop structure, the hairpin loop structure, the multibranched loop structure, the single strand and the end structure of RNA sequence.

14. The predicting program for secondary structure RNA according to any one of claims 11 to 13, wherein said step of calculating sequence energy state is a step of calculating the summation of the free energy of each base constituting said sequence structure state.

15. The predicting program for secondary structure RNA according to any one of claims 11 to 14, wherein said step of arranging the candidate additional stem structure as a defined stem structure is a step of arranging the candidate additional stem structure as a new defined stem structure when an amount of change is negative, the amount of change being obtained by subtracting a sequence energy state of the secondary structure of said RNA sequence in which said defined stem structure is reflected on the secondary structure with a sequence energy state of new secondary structure in which the candidate additional stem structure selected from said candidate stem structure is reflected on the secondary structure.


SEQUENCE LISTING

<110>  NEC Soft, Ltd.

<120>  Method, apparatus and program for predicting a secondary structure of RNA

<130>  10104708

<150>  JP2006-088599
<151>  2006-03-28

<160>  17

<170>  PatentIn version 3.1

<210>  1
<211>  35
<212>  RNA
<213>  Unknown

<220>
<223>  Unknown

<400>  1
ggaaccggug cgcauaacca ccucagugcg agcaa                                35

<210>  2
<211>  38
<212>  RNA
<213>  Unknown

<220>
<223>  Unknown

<400>  2
ggaucccgac uggcgagagc cagguaacga auggaucc                             38

<210>  3
<211>  33
<212>  RNA
<213>  Unknown

<220>
<223>  Unknown

<400>  3
ggaccgucag aggacacggu uaaaaagucc ucu                                  33

<210>  4
<211>  22
<212>  RNA
<213>  Unknown

<220>
<223>  Unknown

<400>  4
ggccgaaauc ccgaaguagg cc                                              22

<210>  5
<211>  33
<212>  RNA
<213>  Unknown

<220>
<223>  Unknown

<400>  5
ggcgauacca gccgaaaggc ccuuggcagc guc                                    33


<210>  6
<211>  29
<212>  RNA
<213>  Unknown

<220>
<223>  Unknown

<400>  6
cauacuugaa acuguaaggu uggcguaug                                         29


<210>  7
<211>  34
<212>  RNA
<213>  Unknown

<220>
<223>  Unknown

<400>  7
gggagcuuga ucccggaaac ggucgaucgc uccc                                   34


<210>  8
<211>  33
<212>  RNA
<213>  Unknown

<220>
<223>  Unknown

<400>  8
ggcgauacca gccgaaaggc ccuuggcagc guc                                    33


<210>  9
<211>  15
<212>  RNA
<213>  Unknown

<220>
<223>  Unknown

<400>  9
ggagaucgca cucca                                                        15


<210>  10
<211>  16
<212>  RNA
<213>  Unknown

<220>
<223>  Unknown

<400>  10
cgaaacauag auucga                                                       16


<210>  11

<211>  20
<212>  RNA
<213>  Unknown

<220>
<223>  Unknown

<400>  11
acuugguuua gguaaugagu                                                    20


<210>  12
<211>  35
<212>  RNA
<213>  Unknown

<220>
<223>  Unknown

<400>  12
ggcguguagg auaugcuucg gcagaaggac acgcc                                   35


<210>  13
<211>  23
<212>  RNA
<213>  Unknown

<220>
<223>  Unknown

<400>  13
ggacugggcg agaaguuuag ucc                                                23


<210>  14
<211>  38
<212>  RNA
<213>  Unknown

<220>
<223>  Unknown

<400>  14
ggaucccgac uggcgagagc cagguaacga auggaucc                                38


<210>  15
<211>  36
<212>  RNA
<213>  Unknown

<220>
<223>  Unknown

<400>  15
gggaagggaa gaaacugcgg cuucggccgg cuuccc                                  36


<210>  16
<211>  27
<212>  RNA
<213>  Unknown

<220>
<223>  Unknown

<400>  16

ggcacgaggu uuagcuacac ucgugcc 27

<210> 17
<211> 15
<212> RNA
<213> Unknown

<220>
<223> Unknown

<400> 17
gcaacccgca uaggg 15

Sequence listing

<110>  NEC Soft Limited

<120>  Method, apparatus and program for predicting a secondary
structure of RNA

<130>  10104708

<150>  JP2006-088599
<151>  2006-03-28

<160>  17

<170>  PatentIn version 3.1

<210>  1
<211>  35
<212>  RNA
<213>  Unknown

<220>
<223>  Unknown

<400>  1
ggaaccggug cgcauaacca ccucagugcg agcaa                                35

<210>  2
<211>  38
<212>  RNA
<213>  Unknown

<220>
<223>  Unknown

<400>  2
ggaucccgac uggcgagagc cagguaacga auggaucc
38

<210>  3
<211>  33
<212>  RNA
<213>  Unknown

<220>
<223>  Unknown

<400>  3
ggaccgucag aggacacggu uaaaaagucc ucu                                    33

<210>  4
<211>  22
<212>  RNA
<213>  Unknown

<220>
<223>  Unknown

<400>  4
ggccgaaauc ccgaaguagg cc                                                22

<210>  5
<211>  33
<212>  RNA
<213>  Unknown

<220>
<223>  Unknown

<400> 5
ggcgauacca gccgaaaggc ccuuggcagc guc 33

<210> 6
<211> 29
<212> RNA
<213> Unknown

<220>
<223> Unknown

<400> 6
cauacuugaa acuguaaggu uggcguaug 29

<210> 7
<211> 34
<212> RNA
<213> Unknown

<220>
<223> Unknown

<400> 7
gggagcuuga ucccggaaac ggucgaucgc uccc 34

<210> 8
<211> 33
<212> RNA
<213> Unknown

<220>
<223> Unknown

<400> 8
ggcgauacca gccgaaaggc ccuuggcagc guc 33

<210> 9
<211> 15
<212> RNA
<213> Unknown

<220>
<223> Unknown

<400> 9
ggagaucgca cucca 15

<210> 10
<211> 16
<212> RNA
<213> Unknown

<220>
<223> Unknown

<400> 10
cgaaacauag auucga 16

<210> 11
<211> 20
<212> RNA
<213> Unknown

<220>
<223> Unknown

<400> 11
acuugguuua gguaaugagu 20

<210> 12
<211> 35
<212> RNA
<213> Unknown

<220>
<223> Unknown

<400> 12
ggcguguagg auaugcuucg gcagaaggac acgcc 35

<210> 13
<211> 23
<212> RNA
<213> Unknown

<220>
<223> Unknown

<400> 13
ggacugggcg agaaguuuag ucc 23

<210> 14
<211> 38
<212> RNA
<213> Unknown

<220>
<223> Unknown

<400> 14
ggaucccgac uggcgagagc cagguaacga auggaucc
38

<210> 15
<211> 36
<212> RNA
<213> Unknown

<220>
<223> Unknown

<400> 15
gggaagggaa gaaacugcgg cuucggccgg cuuccc
36

<210> 16
<211> 27
<212> RNA
<213> Unknown

<220>
<223> Unknown

<400> 16
ggcacgaggu uuagcuacac ucgugcc 27

<210> 17
<211> 15
<212> RNA
<213> Unknown

<220>

<223>  Unknown

<400>  17
gcaacccgca uaggg                                                        15